# EUROPÄISCHE PATENTANMELDUNG

(11) **EP 0 528 415 A1**
(43) Veröffentlichungstag der Anmeldung: **24.02.1993**
(21) Anmeldenummer: 92114143.8
(22) Anmeldetag: 19.08.1992
(51) Int. Cl.: C09K 19/58, G02F 1/1337, C07D 273/00, C07D 285/00, C07D 291/02, C07D 257/02, C07D 259/00, C07D 273/01

(54) **Stabilisierte Komplexliganden und deren Verwendung in Flüssigkristalldisplays**

(30) Priorität: 21.08.1991 DE 4127658
(71) Anmelder: HOECHST AKTIENGESELLSCHAFT, D-65926 Frankfurt am Main (DE)
(72) Erfinder: Manero, Javier, Dr., W-6230 Frankfurt am Main 80 (DE); Rösch, Norbert, Dr., W-6000 Frankfurt am Main 71 (DE)

(57) **Zusammenfassung**

Zersetzungsempfindliche Komplexliganden können stabilisiert werden, indem sie mit Hilfe sperriger sekundärer oder tertiärer Carbon- oder Sulfonsäuren derivatisiert werden. Diese stabilisierten Derivate können Orientierungsschichten und Flüssigkristallmischungen zugegeben werden und bewirken im FLC Display eine Erhöhung des Kontrasts und der Helligkeit.

## Beschreibung

In WO 91/ 08272 wird die Verwendung von Komplexliganden in ferroelektrischen Flüssigkristallmischungen (FLC) bzw. in Orientierungsschichten von Flüssigkristall Displays beschrieben. Mit diesen Stoffen kann das Orientierungs- oder Schaltverhalten der FLC-Mischungen derart beeinflußt werden, daß sich Kontrast und Helligkeit in den Displays erhöhen und die Bildung von Geisterbildern unterdrückt wird.

Es hat sich jedoch im Laufe der Zeit gezeigt, daß sich insbesondere stickstoffhaltige, Komplexliganden unter Einfluß von Licht (UV-Licht) und Wärme zersetzen. Die Zersetzungsprodukte zeichnen sich durch eine gelbe Farbe aus. Werden die stickstoffhaltigen Komplexliganden in Flüssigkristallen gelöst, so führt die geringe Stabilität der Komplexliganden schließlich zu einer Gelbfärbung der Flüssigkristall-Mischung. Ist die Zersetzung des Komplexliganden weit fortgeschritten, so kann letzlich auch die positive Wirkung der Liganden verlorengehen.

Überraschend wurde nun gefunden, daß man diese Stoffe stabilisieren kann, indem sie durch sperrige sekundäre oder tertiäre Carbon- oder Sulfonsäuren derivatisiert werden. Derart derivatisierte Verbindungen zeigen eine gleich gute Wirksamkeit, z. B. auf Unterdrückung von Geisterbildern, wie die nicht derivatisierte Verbindung, und sie haben den Vorteil, daß die Zersetzung und damit eine Gelbfärbung der Flüssigkristallmischung nicht mehr auftritt.

Die Erfindung betrifft somit:
1. Eine Verbindung der allgemeinen Formel I, 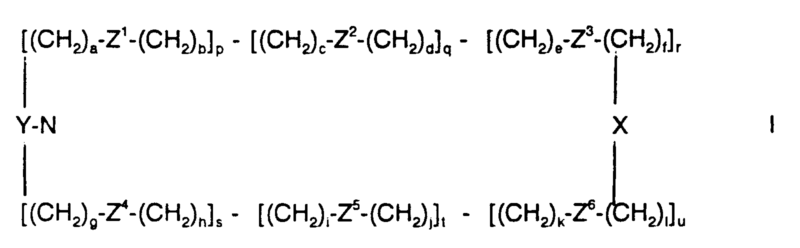 in der
   -Z¹, Z², Z³, Z⁴, z⁵, Z⁶ gleich oder verschieden die Gruppe -O-, -S-, 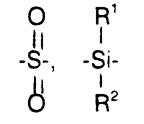 oder 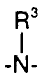 bedeutet,
   oder eine Einfachbindung ist, wobei aber mindestens 3 der Z-Gruppen in der Formel I enthalten sein müssen,
   und wobei R¹ und R² gleich oder verschieden C₁ -C₁₈ Alkyl oder Phenyl, das durch 1 bis 3 C₁-C₄-Alkyl- oder -Alkoxygruppen substituiert sein kann, oder R¹ und R² zusammen -(CH₂)₅- oder -(CH₂)₆- sind und R³ Wasserstoff oder C₁-C₄-Alkyl ist,
   -Y die Gruppe B-(CH₂)ₘ-A- bedeutet, in der 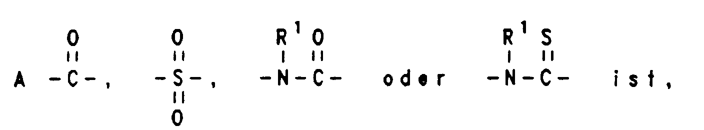 B die Gruppe 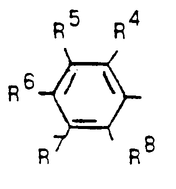 oder 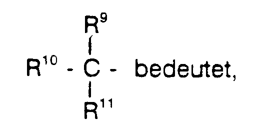 wobei R⁴, R⁵, R⁶, R⁷, R⁸ gleich oder verschieden einen Alkylrest mit 1 bis 5 C-Atomen bedeutet, oder R⁴ und R⁵, R⁵ und R⁶, R⁶ und R⁷ sowie R⁷ und R⁸ jeweils zusammen einen Ring bilden, so daß B einen Naphthalin-, Phenanthren- oder Indenrest darstellt und R⁹, R¹⁰, R¹¹ gleich oder verschieden Alkyl verzweigt oder unverzweigt mit 1 bis 6 C-Atomen bedeutet oder zusammen mit dem tragenden C-Atom ein cyclisches oder polycyclisches System mit 2 bis 12, bevorzugt 2 bis 6 Ringen bildet,

- m die Zahl 0 oder 1 ist,
- x N-Y, -O- oder Z¹ bis Z⁶ ist,
- a, b, c, d, e, f, g, h, i, j, k, l eine Zahl von 0 bis 3 ist, wobei die Summe aus a + b + c + d + e + f + g + h + i + j + k + l bevorzugt 8 bis 16 C-Atomen entspricht, mit der Maßgabe, daß wenn a, f, g und/oder l die Zahl Null sind,
   nur die Gruppen 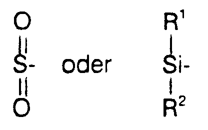 direkt an N oder X gebunden sind, und daß Z¹, Z², Z³ sowie Z⁴, Z⁵, Z⁶ nicht direkt benachbart sind,
   und
- p, q, r, s, t, u die Zahl 0 oder 1 ist, wobei die Summe von p, q, r, s, t, u bevorzugt 2 bis 6 beträgt;
   a, b, c, d, e, f, g, h, i, j, k, l und p, q, r, s, t, u sind so zu wählen, daß sich Ringgrößen von 6 bis 36, bevorzugt von 12 bis 24, ergeben.

Bevorzugt sind Verbindungen der Formel I, in der Y die folgenden Gruppen darstellt:
Adamantylcarbonyl 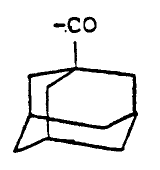
Pivaloyl 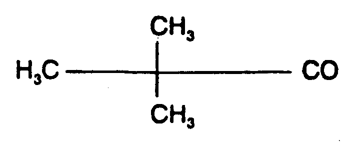
Mesitylencarbonyl 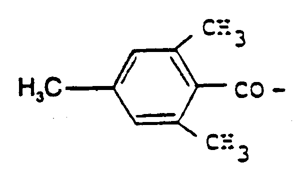
2,6 Dimethylbenzoyl 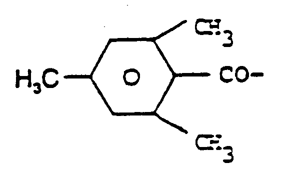
Mesitylensulfonyl 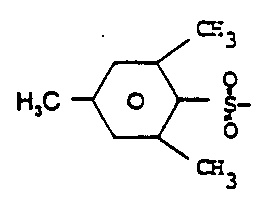
Cubancarbonyl 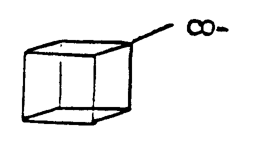
[2.2.2.]-Bicyclooctan-1-carbonyl 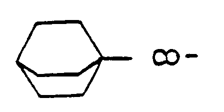
Adamantylacetyl 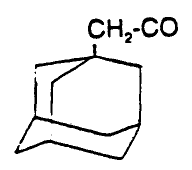
Adamantylthiouryl 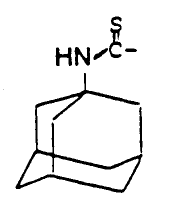
Noradamantylcarbonyl 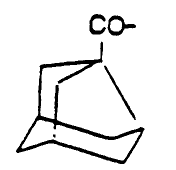
1-Norbornancarbonyl 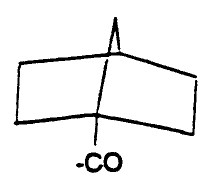
Pagodancarbonyl 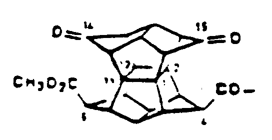
3.3-Dimethylbutyroyl 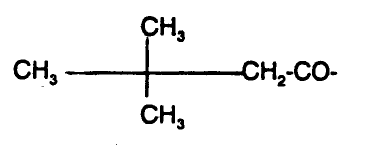
2.2-Dimethylbutyroyl 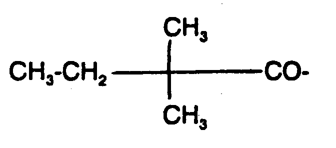
ganz besonders bevorzugt sind Verbindungen der Formel I, in der Y die folgenden Gruppen darstellt:
Adamantylcarbonyl
Pivaloyl
Adamantylacetyl
2,2-Dimethylbutyroyl
3,3-Dimethylbutyroyl
1-Noradamantylcarbonyl
Die Herstellung der Verbindungen der Formel I kann nach den im folgenden zitierten Methoden erfolgen:
1) Houben-Weyl, Methoden der Organischen Chemie, 4. Aufl., Bd. VIII, Sauerstoffverbindungen III, S. 653 bis 671
2) Houben-Weyl, Methoden der Organischen Chemie, 4. Aufl., Bd. E4, Kohlensäurederivate, S. 484 bis 505
3) Houben-Weyl, Methoden der Organischen Chemie, 4. Aufl., Bd. E1, Phosphorverbindungen I, S. 271 bis 313
4) Houben-Weyl, Methoden der Organischen Chemie, 4. Aufl., Bd. E1, Phosphorverbindungen I, S. 313 bis 488
5) Houben-Weyl, Methoden der Organischen Chemie, 4. Aufl., Bd. E2, Phosphorverbindungen II, S. 394 bis 398
6) Houben-Weyl, Methoden der Organischen Chemie, 4. Aufl., Bd. E2, Phosphorverbindungen II, S. 487 bis 831
7) Houben-Weyl, Methoden der Organischen Chemie, 4. Aufl., Bd. E11, Organische Schwefelverbindungen I, S. 655 bis 662
8) Houben-Weyl, Methoden der Organischen Chemie, 4. Aufl., Bd. E11, Organische Schwefelverbindungen II, S. 1.098 bis 1.103
Die Verbindung der Formel I kann ferroelektrischen Flüssigkristallmischungen zugesetzt sowie in oder auf Orientierungsschichten gebracht werden. Die Bindung an die Orientierungsschicht kann über chemische Ankopplung, d. h. Kovalenzbindungen, bzw. durch Physisorption, d. h. durch intermolekulare Anziehungskräfte, an die Orientierungsschichtmoleküle erfolgen. Bei der Physisorption kann die Stärke der Ankopplung an die Orientierungsschichtmoleküle durch Verwendung möglichst polarer Verbindungen der Formel I erhöht werden.

Die Verbindung der allgemeinen Formel I wird in Konzentrationen von 0,01 bis 20 Gew.-%, bevorzugt 0,1 bis 5 Gew.-%, in die Flüssigkristallmischung ein - bzw. in oder auf die Orientierungsschicht aufgebracht.

Die folgenden Beispiele dienen der weitergehenden Erläuterung der Erfindung.

### Beispiele

### Beispiel 1: Synthese von Carbonsäurehalogeniden aus Carbonsäuren

15 ml Carbonsäure werden in 30 bis 100 ml Benzol gelöst und mit 12 ml Oxalylchlorid sowie 2 Tropfen Pyridin versetzt. Nach 18 Std. wird das Solvens abgezogen. Die Probe wird 2 x mit 12 ml Benzol versetzt und nach erneutem Abziehen des Solvens an der Ölpumpe getrocknet.

### Beispiel 2: 4,10-Bis(tert.-butylcarbonyl)-1,7-dioxa-4,10-diazacyclododecan

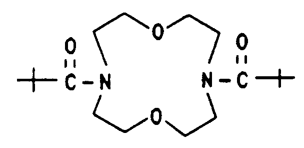

3,81 mmol 1,7-Dioxa-4,10-diazacyclododecan werden in 50 ml CH₂Cl₂ gelöst. Man versetzt mit 1,2 ml Triethylamin und gibt noch 0,1 g DMAP (=4-Dimethylaminopyridin) als Katalysator hinzu. Anschließend fügt man 7,64 mmol Pivalinsäurechlorid zu. Es wird 18 Stunden bei Raumtemparatur gerührt.

Die Reaktionslösung wird 2x mit 1n HCl, anschließend 2x mit gesättigter NaHCO₃-Lösung gewaschen. Man trocknet über MgSO₄ und zieht das Solvens ab. Das Rohprodukt wird säulenchromatographisch an Kieselgel mit CH₂Cl₂/Methanol=20/1 gereinigt.

### Beispiel 3: 4,10-Bis(1-Adamantylcarbonyl)-1,7-dioxa-4,10-diazacyclododecan

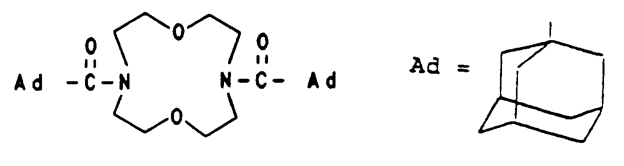

Analog Beispiel 2 aus 1,7-Dioxa-4,10-diazacyclododecan und 1-Adamantylcarbonsäurechlorid.

### Beispiel 4: 4,10-Bis((1-adamanyl)acetyl)-1,7-dioxa-4,10-diazacyclododecan

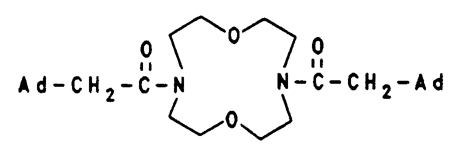

Analog Beispiel 2 aus 1,7-Dioxa-4,10-diazacyclododecan und (1-Adamantyl)essigsäurechlorid.

### Beispiel 5: 7,13-Bis(tert.butylcarbonyl)-1,4,10-trioxa-7,13-diazacyclopentadecan

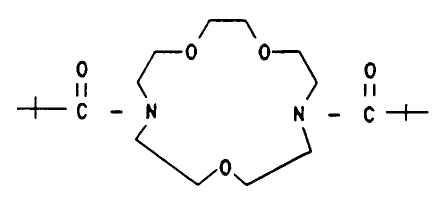

Analog Beispiel 2 aus 1,4,10-Trioxa-7,13-diazacyclopentadecan und Pivalinsäurechlorid.

### Beispiel 6: 7,13-Bis(1-Adamantylcarbonyl)-1,4,10-trioxa-7,13-diazacyclopentadecan

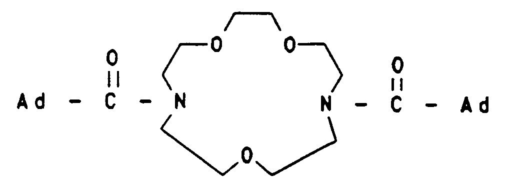

Analog Beispiel 2 aus 1,4,10-Trioxa-7,13-diazacyclopentadecan und 1-Adamantylcarbonsäurechlorid.

### Beispiel 7: 7,13-Bis((1-adamantyl)acetyl)-1,4,10-trioxa-7,13-diazacyclopentadecan

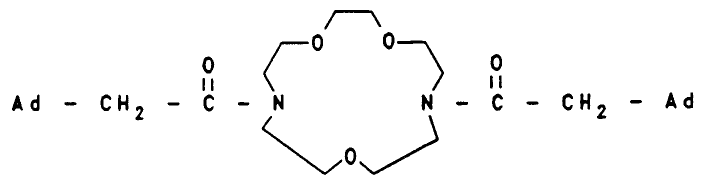

Analog Beispiel 2 aus 1,4,10-Trioxa-7,13-diazacyclopentadecan und (1-Adamantyl)essigsäurechlorid.

### Beispiel 8: 4,13-Bis(tert.-butylcarbonyl)-1,7,10,16-tetraoxa-4,13-diazacyclooctadecan

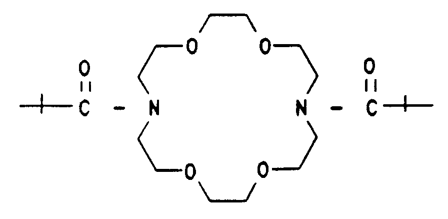

Analog Beispiel 2 aus 1,7,10,16-Tetraoxa-4,13-diazacyclooctadecan und Pivalinsäurechlorid.

### Beispiel 9: 4,13-Bis(1-Adamantylcarbonyl)-1,7,10,16-tetraoxa-4,13-diazacyclooctadecan

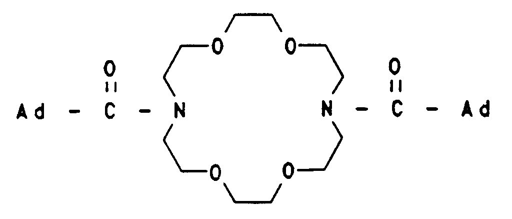

Analog Beispiel 2 aus 1,7,10,16-Tetraoxa-4,13-diazacyclooctadecan und 1-Adamantylcarbonsäurechlorid.

### Beispiel 10: 4,13-Bis((1-adamantyl)acetyl)-1,7,10,16-tetraoxa-4,13-diazacyclooctadecan

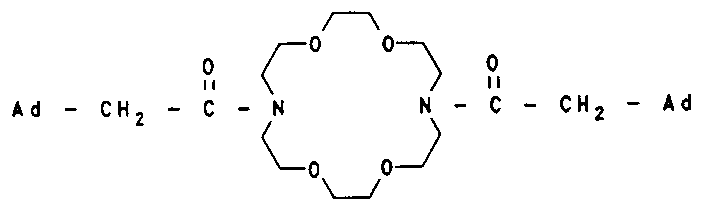

Analog Beispiel 2 aus 1,7,10,16-Tetraoxa-4,13-diazacyclooctadecan und (1-Adamantyl)essigsäurechlorid.

### Beispiel 11: 4,16-Bis(tert.-butylcarbonyl)-1,7,10,13,19-pentaoxa-4,16-diazacycloheneicosan

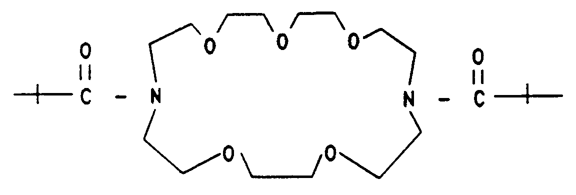

Analog Beispiel 2 aus 1,7,10,13,19-Pentaoxa-4,16-diazacycloheneicosan und Pivalinsäurechlorid.

### Beispiel 12: 4,16-Bis(1-Adamantylcarbonyl)-1,7,10,13,19-pentaoxa-4,16-diazacycloheneicosan

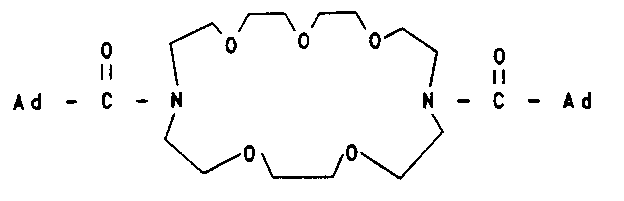

Analog Beispiel 2 aus 1,7,10,13,19-Pentaoxa-4,16-diazacycloheneicosan und 1-Adamantylcarbonsäurechlorid.

### Beispiel 13: 4,16-Bis((1-adamantyl)acetyl)-1,7,10,13,19-pentaoxa-4,16-diazacycloheneicosan

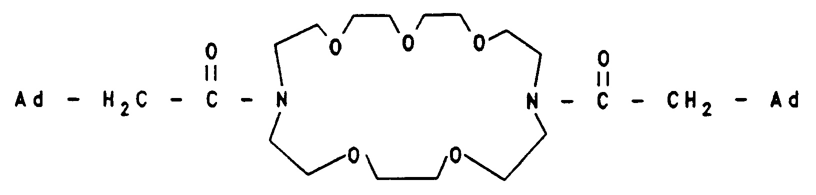

Analog Beispiel 2 aus 1,7,10,13,19-Pentaoxa-4,16-diazacycloheneicosan und (1-Adamantyl)essigsäurechlorid.

### Beispiel 14: 1-(Tert.-butylcarbonyl)-1-aza-4,7,10-trioxacyclododecan

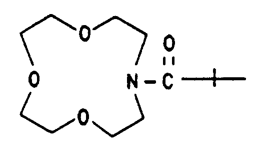

3,81 mmol 1-Aza-4,7,10-trioxacyclododecan werden in 50 ml CH₂Cl₂ gelöst. Man versetzt mit 0,6 ml Triethylamin und gibt noch 0,1 g DMAP (=4-Dimethylaminopyridin) als Katalysator hinzu. Anschließend fügt man 3,82 mmol Pivalinsäurechlorid zu. Es wird 18 Stunden bei Raumtemparatur gerührt.

Die Reaktionslösung wird 2x mit 1n HCl, anschließend 2x mit gesättigter NaHCO₃-Lösung gewaschen. Man trocknet über MgSO₄ und zieht das Solvens ab. Das Rohprodukt wird säulenchromatographisch an Kieselgel mit CH₂Cl₂/Methanol=20/1 gereinigt.

### Beispiel 15: 1-(1-Adamantylcarbonyl)-1-aza-4,7,10-trioxacyclododecan

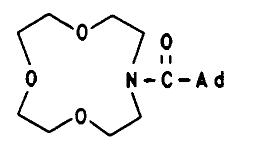

Analog Beispiel 14 aus 1-Aza-4,7,10-trioxacyclododecan und 1-Adamantylcarbonsäurechlorid.

### Beispiel 16: 1-((1-Adamantyl)acetyl)-1-aza-4,7,10-trioxacyclododecan

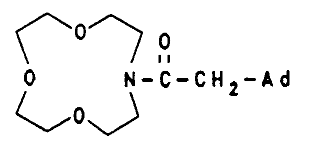

Analog Beispiel 14 aus 1-Aza-4,7,10-trioxacyclododecan und (1-Adamantyl)essigsäurechlorid.

### Beispiel 17: 1-(Tert.-butylcarbonyl)-1-aza-4,7,10,13-tetraoxacyclopentadecan

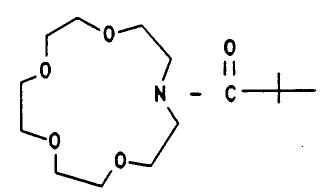

Analog Beispiel 14 aus 1-Aza-4,7,10,13-tetraoxacyclopentadecan und Pivalinsäurechlorid.

### Beispiel 18: 1-(1-Adamantylcarbonyl)-1-aza-4,7,10,13-tetraoxacyclopentadecan

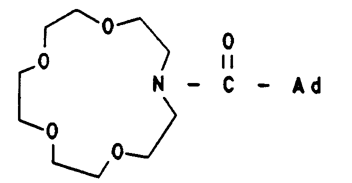

Analog Beispiel 14 aus 1-Aza-4,7,10,13-tetraoxacyclopentadecan und 1-Adamantylcarbonsäurechlorid.

### Beispiel 19: 1-((1-Adamantyl)acetyl)-1-aza-4,7,10,13-tetraoxacyclopentadecan

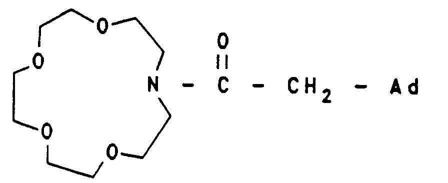

Analog Beispiel 14 aus 1-Aza-4,7,10,13-tetraoxacyclopentadecan und (1-Adamantyl)essigsäurechlorid.

### Beispiel 20: 1-(Tert.-butylcarbonyl)-1-aza-4,7,10,13,16-pentaoxacyclooctadecan

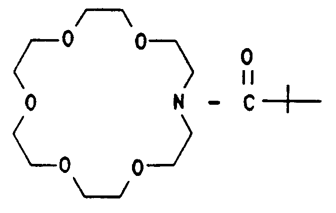

Analog Beispiel 14 aus 1-Aza-4,7,10,13,16-pentaoxacyclooctadecan und Pivalinsäurechlorid.

### Beispiel 21: 1-(1-Adamantylcarbonyl)-1-aza-4,7,10,13,16-pentaoxacyclooctadecan

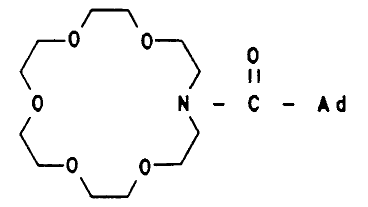

Analog Beispiel 14 aus 1-Aza-4,7,10,13,16-pentaoxacyclooctadecan und 1-Adamantylcarbonsäurechlorid.

### Beispiel 22: 1-((1-Adamantyl)acetyl)-1-aza-4,7,10,13,16-pentaoxacyclooctadecan

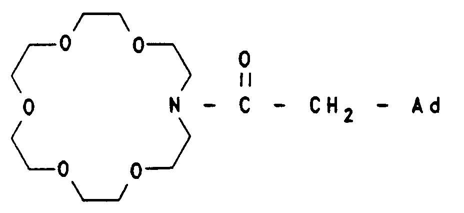

Analog Beispiel 14 aus 1-Aza-4,7,10,13,16-pentaoxacyclooctadecan und (1-Adamantyl)essigsäurechlorid.

### Beispiel 23: 1-(Tert.-butylcarbonyl)-1-aza-4,7,10,13,16,19-hexaoxacycloheneicosan

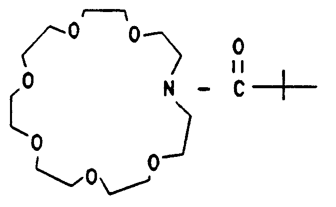

Analog Beispiel 14 aus 1-Aza-4,7,10,13,16,19-hexaoxacycloheneicosan und Pivalinsäurechlorid.

### Beispiel 24: 1-(1-Adamantylcarbonyl)-1-aza-4,7,10,13,16,19-hexaoxacycloheneicosan

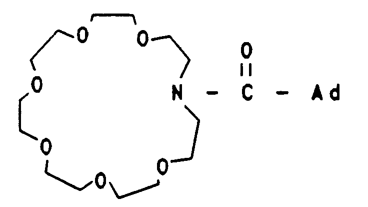

Analog Beispiel 14 aus 1-Aza-4,7,10,13,16,19-hexaoxacycloheneicosan und 1-Adamantylcarbonsäurechlorid.

### Beispiel 25: 1-((1-Adamantyl)acetyl)-1-aza-4,7,10,13,16,19-hexaoxacycloheneicosan

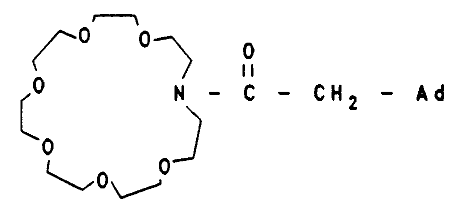

Analog Beispiel 14 aus 1-Aza-4,7,10,13,16,19-hexaoxacycloheneicosan und (1-Adamantyl)essigsäurechlorid.

### Beispiel 26: 4,10-Bis(2,2-dimethylbutyroyl)-1,7-dioxa-4,10-diazacyclododecan

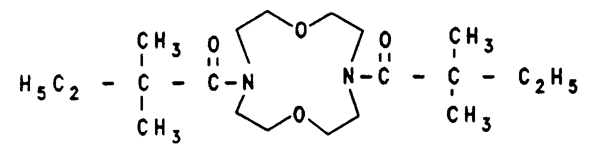

Analog Beispiel 2 aus 1,7-Dioxa-4,10-diazacyclododecan und 2,2-Dimethylbuttersäurechlorid.

### Beispiel 27: 7,13-Bis(2,2-dimethylbutyroyl)-1,4,10-trioxa-7,13-diazacyclopentadecan

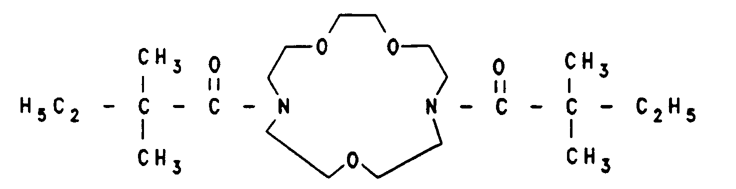

Analog Beispiel 2 aus 1,4,10-Trioxa-7,13-diazacyclopentadecan und 2,2-Dimethylbuttersäurechlorid.

### Beispiel 28: 4,13-Bis(2,2-dimethylbutyroyl)-1,7,10,16-tetraoxa-4,13-diazacyclooctadecan

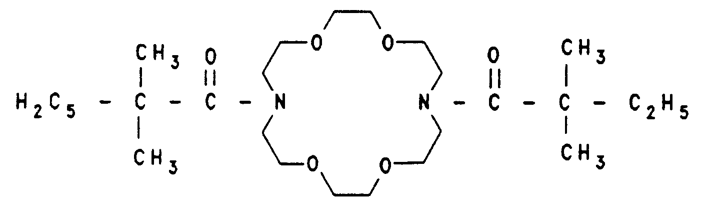

Analog Beispiel 2 aus 1,7,10,16-Tetraoxa-4,13-diazacyclooctadecan und 2,2-Dimethylbuttersäurechlorid.

### Beispiel 29: 4,16-Bis(2,2-dimethylbutyroyl)-1,7,10,13,19-pentaoxa-4,16-diazacycloheneicosan

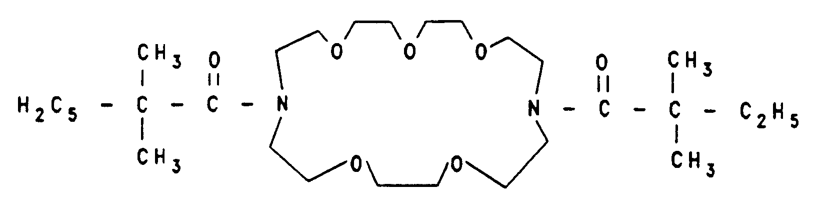

Analog Beispiel 2 aus 1,7,10,13,19-Pentaoxa-4,16-diazacycloheneicosan und 2,2-Dimethylbuttersäurechlorid.

### Beispiel 30: 1-(2,2-Dimethylbutyroyl)-1-aza-4,7,10-trioxacyclododecan

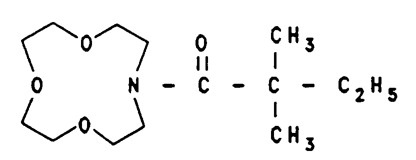

Analog Beispiel 14 aus 1-Aza-4,7,10-trioxacyclododecan und 2,2-Dimethylbuttersäurechlorid.

### Beispiel 31: 1-(2,2-Dimethylbutyroyl)-1-aza-4,7,10,13-tetraoxacyclopentadecan

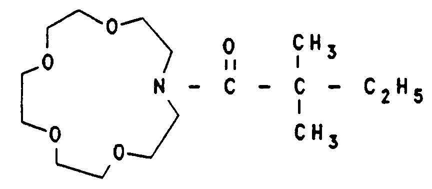

Analog Beispiel 14 aus 1-Aza-4,7,10,13-tetraoxacyclopentadecan und 2,2-Dimethylbuttersäurechlorid.

### Beispiel 32: 1-(2,2-Dimethylbutyroyl)-1-aza-4,7,10,13,16-pentaoxacyclooctadecan

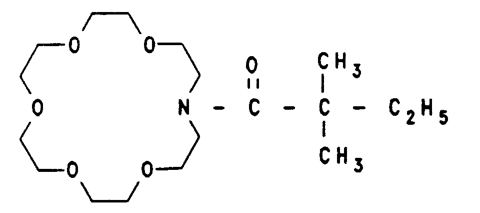

Analog Beispiel 14 aus 1-Aza-4,7,10,13,16-pentaoxacyclooctadecan und 2,2-Dimethylbuttersäurechlorid.

### Beispiel 33: 1-(2,2-Dimethylbutyroyl)-1-aza-4,7,10,13,16,19-hexaoxacycloheneicosan

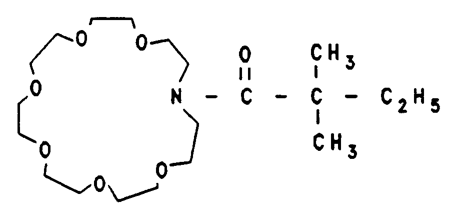

Analog Beispiel 14 aus 1-Aza-4,7,10,13,16,19-hexaoxacycloheneicosan und 2,2-Dimethylbuttersäurechlorid.

### Beispiel 34: 4,10-Bis(3,3-dimethylbutyroyl)-1,7-dioxa-4,10-diazacyclododecan

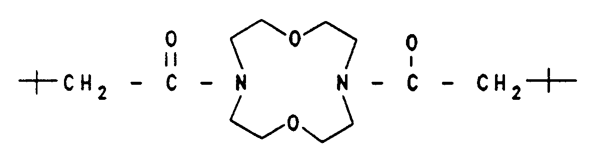

Analog Beispiel 2 aus 1,7-Dioxa-4,10-diazacyclododecan und 3,3-Dimethylbuttersäurechlorid.

### Beispiel 35: 7,13-Bis(3,3-dimethylbutyroyl)-1,4,10-trioxa-7,13-diazacyclopentadecan

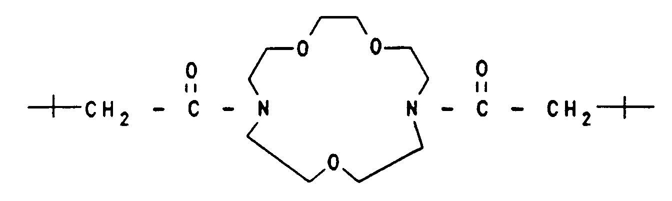

Analog Beispiel 2 aus 1,4,10-Trioxa-7,13-diazacyclopentadecan und 3,3-Dimethylbuttersäurechlorid.

### Beispiel 36: 4,13-Bis(3,3-dimethylbutyroyl)-1,7,10,16-tetraoxa-4,13-diazacyclooctadecan

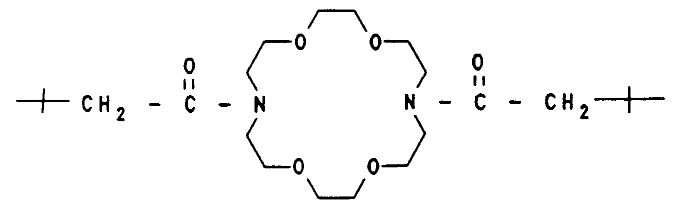

Analog Beispiel 2 aus 1,7,10,16-Tetraoxa-4,13-diazacyclooctadecan und 3,3-Dimethylbuttersäurechlorid.

### Beispiel 37: 4,16-Bis(3,3-dimethylbutyroyl)-1,7,10,13,19-pentaoxa-4,16-diazacycloheneicosan

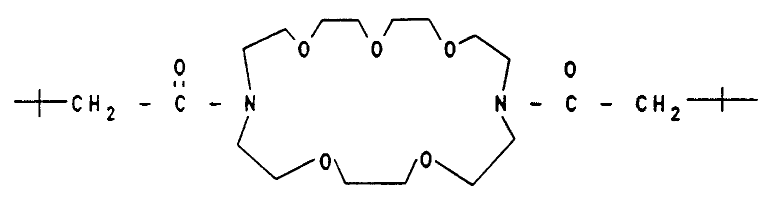

Analog Beispiel 2 aus 1,7,10,13,19-Pentaoxa-4,16-diazacycloheneicosan und 3,3-Dimethylbuttersäurechlorid.

### Beispiel 38: 1-(3,3-Dimethylbutyroyl)-1-aza-4,7,10-trioxacyclododecan

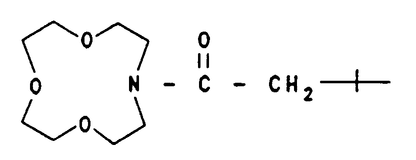

Analog Beispiel 14 aus 1-Aza-4,7,10-trioxacyclododecan und 3,3-Dimethylbuttersäurechlorid.

### Beispiel 39: 1-(3,3-Dimethylbutyroyl)-1-aza-4,7,10,13-tetraoxacyclopentadecan

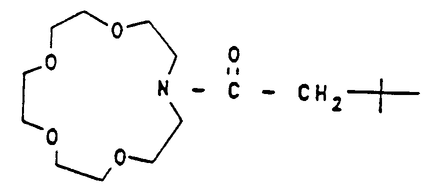

Analog Beispiel 14 aus 1-Aza,-4,7,10,13-tetraoxacyclopentadecan und 3,3-Dimethylbuttersäurechlorid.

### Beispiel 40: 1-(3,3-Dimethylbutyroyl)-1-aza-4,7,10,13,16-pentaoxacyclooctadecan

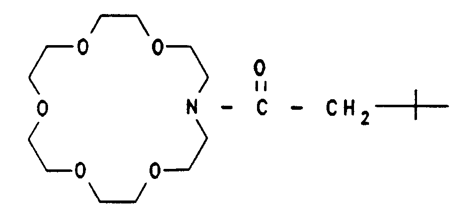

Analog Beispiel 14 aus 1-Aza-4,7,10,13,16-pentaoxacyclooctadecan und 3,3-Dimethylbuttersäurechlorid.

### Beispiel 41: 1-(3,3-Dimethylbutyroyl)-1-aza-4,7,10,13,16,19-hexaoxacycloheneicosan

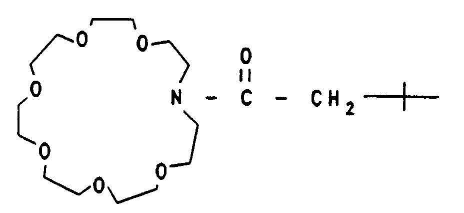

Analog Beispiel 14 aus 1-Aza-4,7,10,13,16,19-hexaoxacycloheneicosan und 3,3-Dimethylbuttersäurechlorid.

### Beispiel 42: 4,10-Bis(1-noradamantylcarbonyl)-1,7-dioxa-4,10-diazacyclododecan

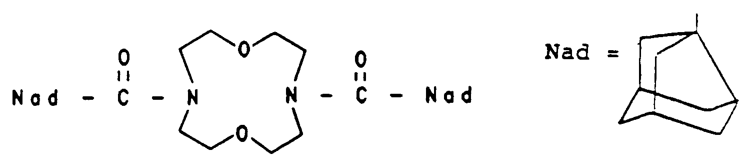

Analog Beispiel 2 aus 1,7-Dioxa-4,10-diazacyclododecan und 1-Noradamantancarbonsäurechlorid.

### Beispiel 43: 7,13-Bis(1-noradamantylcarbonyl)-1,4,10-trioxa-7,13-diazacyclopentadecan

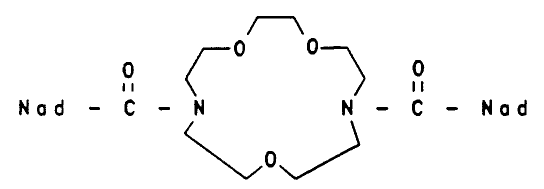

Analog Beispiel 2 aus 1,4,10-Trioxa-7,13-diazacyclopentadecan und 1-Noradamantancarbonsäurechlorid.

### Beispiel 44: 4,13-Bis(1-noradamantylcarbonyl)-1,7,10,16-tetraoxa-4,13-diazacyclooctadecan

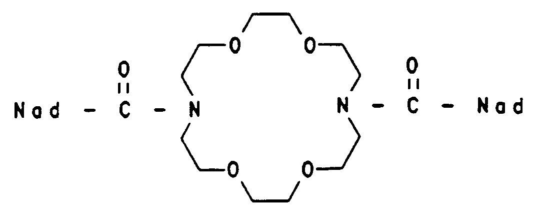

Analog Beispiel 2 aus 1,7,10,16-Tetraoxa-4,13-diazacyclooctadecan und 1-Noradamantancarbonsäurechlorid.

### Beispiel 45: 4,16-Bis(1-noradamantylcarbonyl)-1,7,10,13,19-pentaoxa-4,16-diazacycloheneicosan

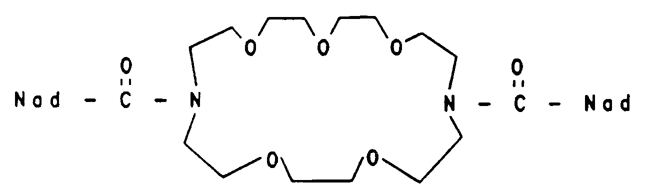

Analog Beispiel 2 aus 1,7,10,13,19-Pentaoxa-4,16-diazacycloheneicosan und 1-Noradamantancarbonsäurechlorid.

### Beispiel 46: 1-(1-Noradamantylcarbonyl)-1-aza-4,7,10-trioxacyclododecan

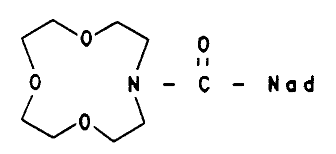

Analog Beispiel 14 aus 1-Aza-4,7,10-trioxacyclododecan und 1-Noradamantancarbonsäurechlorid.

### Beispiel 47: 1-(1-Noradamantylcarbonyl)-1-aza-4,7,10,13-tetraoxacyclopentadecan

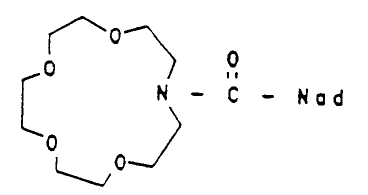

Analog Beispiel 14 aus 1-Aza-4,7,10,13-tetraoxacyclopentadecan und 1-Noradamantancarbonsäurechlorid.

### Beispiel 48: 1-(1-Noradamantylcarbonyl)-1-aza-4,7,10,13,16-pentaoxacyclooctadecan

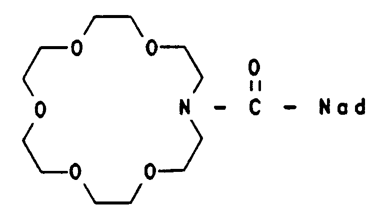

Analog Beispiel 14 aus 1-Aza-4,7,10,13,16-pentaoxacyclooctadecan und 1-Noradamantancarbonsäurechlorid.

### Beispiel 49: 1-(1-Noradamantylcarbonyl)-1-aza-4,7,10,13,16,19-hexaoxacycloheneicosan

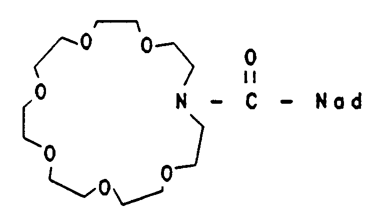

Analog Beispiel 14 aus 1-Aza-4,7,10,13,16,19-hexaoxacycloheneicosan und 1-Noradamantancarbonsäurechlorid.

### Beispiel 50: Anwendungsbeispiel - Stabilitätsnachweis

Zum Nachweis der erhöhten chemischen Stabilität der erfindungsgemäßen Verbindungen wurde Z1 und das als lichtempfindlich bekannte Kryptofix 22 in eine flüssigkristalline Mischung gegeben.

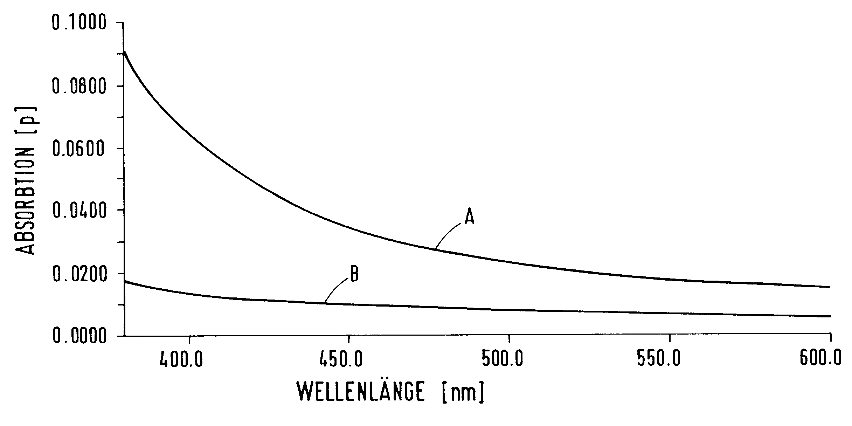

Beide Proben wurden bei 50°C mit einer UV-Lampe (Sun Tester der Firma Heraeus) 30 Min. bestrahlt. Anschließend wurden die bestrahlten Proben in Methylenchlorid gelöst und die entsprechenden optischen Spektren mit einem Photometer aufgenommen. Als Referenz diente der reine, in Methylenchlorid gelöste Flüssigkristall.
Z1: 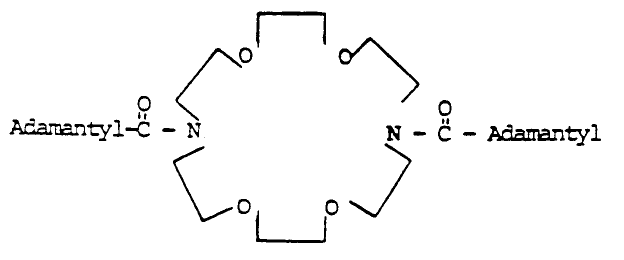
Kryptofix 22 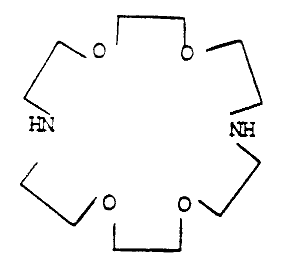
Die Abbildung zeigt die optischen Spektren der Flüssigkristallmischung mit Kryptofix 22 (Kurve A) und der Mischung mit Z1 (Kurve B). Die bei Zersetzung von Kryptofix 22 entstehende Gelbfärbung der Probe führt im Spektrum zu einer Absorption im Bereich zwischen 400 bis 500 nm. In diesem Bereich zeigt die Mischung mit der erfindungsgemäßen Verbindung keine nennenswerte Absorption.

## Patentansprüche

1. Verbindung der allgemeinen Formel I 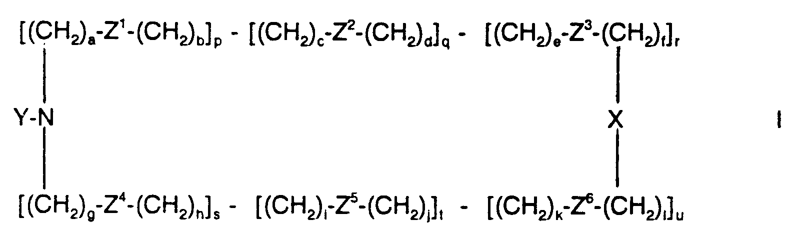 in der
-Z¹, Z², Z³, Z⁴, Z⁵, Z⁶ gleich oder verschieden die Gruppe -O-, -S-, 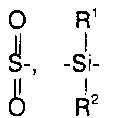 oder 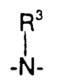 bedeutet,
oder eine Einfachbindung ist, wobei aber mindestens 3 der Z-Gruppen in der Formel I enthalten sein müssen,
und wobei R¹ und R² gleich oder verschieden C₁ -C₁₈ Alkyl oder Phenyl, das durch 1 bis 3 C₁-C₄-Alkyl- oder -Alkoxygruppen substituiert sein kann, oder R¹ und R² zusammen -(CH₂)₅- oder -(CH₂)₆- sind und R³ Wasserstoff oder C₁-C₄-Alkyl ist,
- Y die Gruppe B-(CH₂)ₘ-A-bedeutet, in der 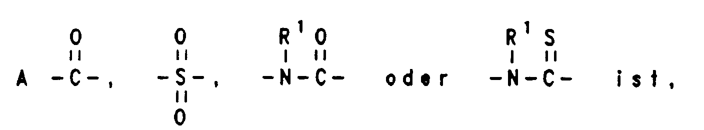 B die Gruppe 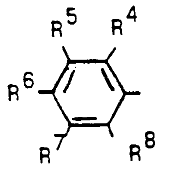 oder 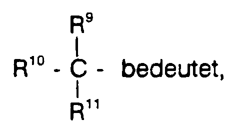 wobei R⁴, R⁵, R⁶, R⁷, R⁸ gleich oder verschieden einen Alkylrest mit 1 bis 5 C-Atomen bedeutet, oder R⁴ und R⁵, R⁵ und R⁶, R⁶ und R⁷ sowie R⁷ und R⁸ jeweils zusammen einen Ring bilden, so daß B einen Naphthalin-, Phenanthren- oder Indenrest darstellt und R⁹, R¹⁰, R¹¹ gleich oder verschieden Alkyl verzweigt oder unverzweigt mit 1 bis 6 C-Atomen bedeutet oder zusammen mit dem tragenden C-Atom ein cyclisches oder polycyclisches System mit 2 bis 12, bevorzugt 2 bis 6 Ringen bildet,
- m die Zahl 0 oder 1 ist,
- X N-Y, -O- oder Z¹ bis Z⁶ ist,
- a, b, c, d, e, f, g, h, i, j, k, l eine Zahl von 0 bis 3 ist, wobei die Summe aus a + b + c + d + e + f + g + h + i + j + k + l bevorzugt 8 bis 16 C-Atomen entspricht, mit der Maßgabe, daß wenn a, f, g und/oder I die Zahl Null sind,
nur die Gruppen 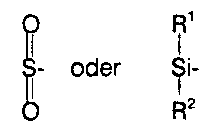 direkt an N oder X gebunden sind, und daß Z¹, Z², Z³ sowie Z⁴, Z⁵, Z⁶ nicht direkt benachbart sind,
und
- p, q, r, s, t, u die Zahl 0 oder 1 ist, wobei die Summe von p q, r s, t, u bevorzugt 2 bis 6 beträgt;
a, b, c, d, e, f, g, h, i, j, k, l und p, q, r, s, t, u sind so zu wählen, daß sich Ringgrößen von 6 bis 36, bevorzugt von 12 bis 24, ergeben.

2. Verfahren zur Stabilisierung von Komplexliganden, dadurch gekennzeichnet, daß die Komplexliganden mit sperrigen sekundären oder tertiären Carbonsäuren und/oder Suffonsäuren derivatisiert werden.

3. Verwendung der Verbindungen der allgemeinen Formel I nach Anspruch 1 als Zusatz zu Flüssigkristallmischungen.

4. Verwendung der Verbindung der allgemeinen Formel I nach Anspruch 1 als Zusatz in Orientierungsschichten.

5. Verwendung nach Anspruch 3 oder 4 dadurch gekennzeichnet, daß die Verbindung der Formel I in Konzentrationen von 0,01 bis 20 Gew.-% eingesetzt wird.

6. Flüssigkristallmischungen, gekennzeichnet durch einen Gehalt von 0.01 bis 20 Gew.-% an mindestens einer Verbindung der Formel I nach Anspruch 1.

7. Orientierungsschicht, gekennzeichnet durch einen Gehalt von 0.01 bis 20 Gew.-% an mindestens einer Verbindung der Formel I nach Anspruch 1.

8. Elektrooptisches Schalt- und Anzeigeelement, enthaltend die Flüssigkristallmischung und/oder die Orientierungsschicht nach Anspruch 6 oder 7.
